# EUROPÄISCHE PATENTSCHRIFT

(11) **EP 3 719 476 B1**
(45) Veröffentlichungstag und Bekanntmachung des Hinweises auf die Patenterteilung: **11.01.2023**
(21) Anmeldenummer: 20167758.0
(22) Anmeldetag: 02.04.2020
(51) Int. Cl.: G01N 15/06, G01N 15/14, G01N 33/18, G01N 15/00

(54) **ROHRLEITUNG MIT EINER MESSVORRICHTUNG ZUR ERFASSUNG VON PARTIKELN UND VORRICHTUNG ZUR ÜBERMITTLUNG VON DATEN AUS EINER ROHRLEITUNG**
PIPELINE COMPRISING MEASURING DEVICE FOR DETECTING PARTICLES AND APPARATUS FOR TRANSMITTING DATA OUT OF A PIPELINE
CONDUITE COMPRENANT DISPOSITIF DE MESURE PERMETTANT DE DÉTECTER DES PARTICULES ET DISPOSITIF POUR LA TRANSMISSION DE DATES DEPUIS LA CONDUITE

(30) Priorität: 02.04.2019 DE 102019204668
(43) Veröffentlichungstag der Anmeldung: 07.10.2020
(73) Patentinhaber: QUNDIS GmbH, 99098 Erfurt (DE)
(72) Erfinder: DÖBEL, Christian, 99880 Waltershausen (DE)
(74) Vertreter: Liedtke & Partner Patentanwälte

(56) Entgegenhaltungen:
- WO-A1-2010/051842
- WO-A1-2018/001627
- US-A1- 2017 219 550
- US-A1- 2017 242 234
- Anonymous: "Totleitung - Wikipedia", , 31. Dezember 2018 (2018-12-31), XP055718005, Gefunden im Internet: URL:https://de.wikipedia.org/wiki/Totleitu ng [gefunden am 2020-07-24]

## Beschreibung

Die Erfindung betrifft eine Rohrleitung mit einer Messvorrichtung, insbesondere eine Sensoreinheit, zur Erfassung von Partikeln in einer Rohrleitung. Des Weiteren betrifft die Erfindung eine Vorrichtung zur Übermittlung von Daten aus einer Rohrleitung mit einer solchen Messvorrichtung.

Fluidtransportsysteme, insbesondere Rohrleitungssysteme, wie zum Beispiel Wasserversorgungsanlagen und/oder Trinkwasseranlagen versorgen täglich eine Vielzahl von Wohneinheiten mit Trinkwasser. In Abhängigkeit von unterschiedlichen, gesundheitsschädlichen Partikeln im Trinkwasser können Personen erkranken.

Aus dem Stand der Technik sind Messvorrichtungen bekannt, die zur Prüfung der Wasser- und/oder Trinkwasserqualität eine Probeentnahme benötigen. Für die Probeentnahme muss eine Wasserzufuhr verriegelt und die Rohrleitung an einer bestimmten Stelle zur Probeentnahme geöffnet werden. Andere Messvorrichtungen werden in verifizierten Laboren (nach Trinkwasserschutzverordnung) bereitgestellt, die zur Prüfung der Wasser- und/oder Trinkwasserqualität ebenfalls eine Probeentnahme benötigen. Die Probe wird an das Labor übermittelt, welche mittels geeigneter Messvorrichtungen die Wasser- und/oder Trinkwasserqualität prüfen.

Beispielsweise sind Vorrichtungen und Verfahren zur Ermittlung von Partikeln in einem Fluid aus der WO 2010/051842 A1, der WO 2018/001627 A1, der US 2017/219550 A1 und der US 2017/242234 A1 bekannt.

Der Erfindung liegt die Aufgabe zu Grunde, eine gegenüber dem Stand der Technik verbesserte Messvorrichtung zur Erfassung von Partikeln in einer Rohrleitung anzugeben.

Hinsichtlich der Rohrleitung mit einer Messvorrichtung wird die Aufgabe erfindungsgemäß durch die im Anspruch 1 angegebenen Merkmale gelöst. Hinsichtlich der Vorrichtung zur Übermittlung von Daten aus einer Rohrleitung wird die Aufgabe durch die im Anspruch 12 angegebenen Merkmale gelöst.

Vorteilhafte Ausgestaltungen der Erfindung sind Gegenstand der Unteransprüche.

Die Messvorrichtung zur Erfassung von Partikeln in einer Rohrleitung umfasst zumindest eine Erfassungseinheit, die in einem Rohrinnenraum der Rohrleitung angeordnet ist, wobei der Rohrinnenraum ein Medium, wie beispielsweise Wasser und/oder Trinkwasser, aufweist und/oder mit einem Medium durchströmbar ist, wobei die Erfassungseinheit ausgebildet ist, zumindest ein Auftreten unterschiedlicher Partikel im Medium zu erfassen. Beispielsweise ist der Rohrinnenraum mit einem Medium, wie beispielsweise Wasser und/oder Trinkwasser, befüllt. Des Weiteren kann der Rohrinnenraum mit einem Medium, wie Wasser und/oder Trinkwasser, zur Weiterleitung des Mediums durchströmt werden.

Beispielsweise ist die Messvorrichtung teilweise oder vollständig in der Rohrleitung integriert, insbesondere im Rohrinnenraum fixiert. Die Rohrleitung ist beispielsweise Teil eines Fluidtransportsystems, wie beispielsweise eines Wasserversorgungssystems. Das Wasserversorgungssystem, wie beispielsweise eine Wasserversorgungsanlage, stellt Wasser und/oder Trinkwasser für zumindest eine Wohneinheit, beispielsweise Wohnung oder Haus, bereit. Die Rohrleitung kann selbst ein Fluidtransportsystem sein. Die Erfassungseinheit ist beispielsweise ein Messgerät.

Die Messvorrichtung ist ausgebildet, um das Medium, wie beispielsweise Wasser und/oder Trinkwasser, kontinuierlich und/oder in vorgebbaren Zeitabständen auf gesundheitsschädliche Partikel zu prüfen. Insbesondere können Partikel, insbesondere schädliche Fremdstoffe, im Medium frühzeitig erkannt werden.

Beispielsweise werden nicht sichtbare, insbesondere für Menschen, gesundheitsschädliche Partikel im Medium mitgeführt, die die Hygiene des Mediums beeinträchtigen. Die Erfassungseinheit umfasst beispielsweise eine Steuereinheit, mittels welche die Erfassungseinheit programmierbar ist. Beispielsweise ist die Erfassungseinheit von außen ansteuerbar, beispielsweise einstellbar, konfigurierbar. Die Erfassungseinheit kann auch bereits im Werk eingestellt bzw. konfiguriert werden. Die Erfassungseinheit kann auch nach Installation in der Rohrleitung eingestellt bzw. konfiguriert werden. Die Erfassungseinheit muss zur Einstellung derselben nicht aus der Rohrleitung demontiert werden. Die Erfassungseinheit weist beispielsweise eine Anzahl von Schnittstellen, wie Kommunikationsschnittstellen, insbesondere drahtlose Kommunikationsschnittstellen auf. Über eine oder mehrere Schnittstellen kann die Erfassungseinheit mit einem zentralen Server verbunden werden. Über eine oder mehrere Schnittstellen kann die Erfassungseinheit mit einem mobilen Gerät eines Nutzers verbunden werden.

Vorteile der Erfindung bestehen darin, dass der Rohrinnenraum zu jeder Zeit nach gesundheitsschädlichen oder anderen, zu prüfenden Partikeln prüfbar ist, wobei aufwändige, herkömmliche Probeentnahmen entfallen. Insbesondere sind ein Zeitaufwand und ein Kostenaufwand zur Prüfung des Mediums verringert. Zudem ist beispielsweise eine Tendenz zur Zunahme einer Partikelkonzentration gesundheitsschädlicher Partikel frühzeitig erkennbar. Die Erfassungseinheit ist beispielsweise für Wartungsarbeiten lösbar im Rohrinnenraum fixiert. Insbesondere erfolgt eine Datenübertragung drahtlos. Zum Beispiel ist die Erfassungseinheit ausgebildet, mit stationären oder mobilen Datenverarbeitungseinheiten und/oder Datensammlern zu kommunizieren. Beispielsweise weist die Erfassungseinheit eine Anzahl von Schnittstellen auf. Mittels der Anordnung der Erfassungseinheit direkt im Rohrinnenraum ist dieser kontinuierlich überwachbar. Die Erfassungseinheit ist beispielsweise ausgebildet, in vorgebbaren Zeitabständen Daten zum Zustand des Mediums zu erfassen. Das in der Rohrleitung eines Wasserversorgungssystems transportierte Medium kann insbesondere gegenüber dem Stand der Technik in unkomplizierter Weise auf Hygiene geprüft werden.

Die Messvorrichtung ist beispielsweise als ein Montagemodul an einer geeigneten Mediumdurchflussstelle oder Mediumstehstelle innerhalb der Rohrleitung, wie einer Wasserleitung, angeordnet und montiert, beispielsweise verschraubt. Die Erfassungseinheit ist beispielsweise eine Sensoreinheit. Da die Erfassungseinheit innerhalb der Rohrleitung angeordnet und montiert ist, ist keine zusätzliche und zeitaufwendige Probeentnahme benötigt. Die Erfassungseinheit prüft das Medium beispielsweise während es durch die Rohrleitung durchfließt oder in der Rohrleitung steht, ob gefährliche und/oder gesundheitsschädliche biologische Partikel, wie Legionellen im Medium, insbesondere Wasser vorhanden sind.

In einer Weiterbildung ist die Erfassungseinheit ausgebildet, biologische Partikel im Wasser und/oder Trinkwasser zu klassifizieren und zu quantifizieren. Zum Beispiel werden unter biologischen Partikeln Pilze und Bakterien, wie Kolibakterien (Escherichia coli, kurz auch E.coli), Legionellen oder andere gesundheitsschädliche Partikel und/oder Schädlinge verstanden. Darüber hinaus ist es möglich, das Medium auf andere Schadstoffe, wie Korrosionsprodukte, zu überprüfen. Insbesondere sind im Medium mitgeführte Partikel mittels der Erfassungseinheit erfassbar. Beispielsweise ist die Erfassungseinheit ausgebildet, Partikelarten zu klassifizieren. Des Weiteren ist die Erfassungseinheit ausgebildet, einen Partikelanteil (Volumenanteil), eine Partikelkonzentration (Volumenkonzentration), ein Volumenverhältnis beispielsweise in Volumenprozent in Bezug auf ein Volumen des Mediums und/oder eine Partikeldichte und/oder einen Massenanteil zu ermitteln.

Zum Beispiel ist die Erfassungseinheit konfiguriert, zur Quantifizierung der Partikel die so genannte koloniebildende Einheit (kurz: KBE) zu verwenden. Grenz- und Richtwerte, beispielsweise für mikrobiologische Parameter, sind in der Trinkwasserverordnung (TrinkwV) aufgeführt. Weiterhin ist die Erfassungseinheit konfiguriert, eine statistische Verteilung der Partikel im Medium zu ermitteln. Beispielsweise umfasst die Erfassungseinheit eine Auswerteeinheit. Die Auswerteeinheit ist in Form einer Auswerteelektronik, umfassend eine Software und Algorithmen, in die Erfassungseinheit eingebettet. Dadurch können herkömmliche Schritte, wie zum Beispiel Abriegeln der Rohrleitung und/oder einer Mediumzufuhr, Entnahme des Mediums, Bestücken eines Testgeräts, entfallen. Darüber hinaus ist die in der Rohrleitung angeordnete und beispielsweise integrierte Erfassungseinheit ausgebildet, bei Ansteuerung zur Durchführung einer Prüfung das Medium unmittelbar zu prüfen. Daten über das Medium können also in Echtzeit permanent erfasst und übertragen werden. Dies führt zu einem schnellen Ergebnis mit geringem Zeitaufwand gegenüber herkömmlichen Maßnahmen. Zudem ist eine "Online- Überwachung und Datenübertragung" möglich.

Beispielsweise ist die Erfassungseinheit in einem Netzwerk, wie einem Kommunikationsnetzwerk eingebunden und kann über zumindest eine Kommunikationsschnittstelle mit externen und/oder zentralen Servern und Geräten kommunizieren. Zum Beispiel ist es möglich, rein erfasste und noch nicht ausgewertete Daten und/oder bereits durch die Erfassungseinheit, insbesondere die in der Erfassungseinheit integrierte Auswerteeinheit ermittelte, d.h. ausgewertete Daten zu übermitteln. Beispielsweise werden die Daten an eine Datenverarbeitungseinheit, wie beispielsweise ein mobiles Endgerät eines Nutzers, und/oder an einen zentralen Server, einer Cloud und/oder an ein Labor übermittelt. Zum Beispiel sendet die Erfassungseinheit die Daten, wenn die Erfassungseinheit über eine drahtlose Kommunikationsschnittstelle eine vom zentralen Server und/oder vom externen Gerät ausgesendete Anforderung empfängt.

In einer weiteren Ausführungsform sind die Daten mit einem Zeitstempel für weitere zeitabhängige Analysen und statistische Verarbeitung der Daten versehen.

Gemäß einer weiteren Ausführungsform ist die Erfassungseinheit ausgebildet, von einem flüssigen Medium durchströmt zu werden. Alternativ oder zusätzlich ist die Erfassungseinheit ausgebildet, im Medium zu stehen. Beispielsweise ist die Erfassungseinheit im Wesentlichen kreisförmig ausgebildet. Zum Beispiel korrespondiert eine Abmessung, wie ein Durchmesser, der Erfassungseinheit mit einem Querschnitt der Rohrleitung. Am Außenumfang der Erfassungseinheit sind beispielsweise lösbare Fixierungselemente angeordnet. Im Rohrinnenraum sind mit den lösbaren Fixierungselementen der Erfassungseinheit korrespondierende Fixierungspunkte ausgebildet. Zum Beispiel ist die Erfassungseinheit ringförmig ausgebildet. Die Erfassungseinheit umfasst eine oder mehrere Öffnung/en, durch welche das Medium durchströmen kann.

Die Erfassungseinheit ist beispielsweise gegenüber dem Querschnitt der Rohrleitung kleiner ausgebildet. Beispielsweise ist die Erfassungseinheit an einem Innenwandungsabschnitt der Rohrleitung befestigt. Zum Beispiel ist die Erfassungseinheit am Innenwandungsabschnitt lösbar fixiert.

In einer Weiterbildung ist die Erfassungseinheit Stoff-, kraft- und/oder formschlüssig mit dem Rohrinnenraum verbunden.

Eine weitere mögliche Ausgestaltung sieht vor, dass die Erfassungseinheit in einem Steigleitungsabschnitt oder einem Totleitungsabschnitt der Rohrleitung angeordnet ist.

Die Erfassungeinheit umfasst eine Filtereinheit, die dazu ausgebildet ist, die zu erfassenden Partikel mit einer Fluoreszenzmarkierung zu versehen.

In einer weiteren Ausgestaltung ist die Erfassungseinheit ausgebildet, fluoreszierende Partikel zu erfassen.

Beispielsweise umfasst die Erfassungseinheit eine Mehrzahl von Teilchen, beispielsweise geeigneten Antikörpern, die mit unterschiedlichen Partikeln reagieren. Insbesondere sind die Partikel durch die Teilchen anregbar, sodass die Partikel fluoreszieren. Eine chemische Reaktion zwischen Partikel und Antikörper bewirkt also eine von der Erfassungseinheit erfassbare Fluoreszenz. Beispielsweise ist eine Biofluoreszenz mittels der Erfassungseinheit erfassbar.

Gemäß einer Ausführungsform umfasst die Erfassungseinheit zumindest eine Kameraeinheit. Die Kameraeinheit ist ausgebildet, fluoreszierende Partikel zu erfassen. Beispielsweise ist die Kameraeinheit konfiguriert, die fluoreszierenden Partikel im sogenannten VIS-Bereich zu erfassen.

Die Erfassungseinheit umfasst zumindest eine Filtereinheit, die ausgebildet ist, zu erfassende Partikel mit einer Fluoreszenzmarkierung zu versehen. Beispielsweise sind in der Filtereinheit spezielle Teilchen, wie Antikörper, angeordnet, die die insbesondere gesundheitsschädlichen Partikel zum Fluoreszieren bringen. Insbesondere ist die Filtereinheit ausgebildet, vom Medium durchströmt zu werden und die im Medium mitgeführten gesundheitsschädlichen Partikel mit einer Fluoreszenzmarkierung zu versehen.

In einer möglichen weiteren Ausbildung sind die Kameraeinheit und die Filtereinheit in Form eines Messumformers ausgebildet. Beispielsweise umfasst die Erfassungseinheit einen Messumformer sowie einer Auswerteelektronik mit Software.

In einer möglichen Ausgestaltung ist die Erfassungseinheit zumindest teilweise lösbar im Rohrinnenraum fixiert. Dadurch können Wartungsarbeiten, Reparaturarbeiten sowie ein Austausch von in der Erfassungseinheit integrierten Komponenten in einfacher Weise durchgeführt werden. Beispielsweise ist die Filtereinheit in einem Nebenpfad der Rohrleitung, insbesondere eines Medienflusses, angeordnet, wobei der Nebenpfad zum Zweck von Wartungsarbeiten, Auswechslung und/oder Reinigung der Filtereinheit vom Medium abgeriegelt werden kann. Des Weiteren ist beispielsweise die Kameraeinheit zum Zweck von Wartungsarbeiten und/oder Reinigung jederzeit entfernbar.

Eine weitere mögliche Ausgestaltung sieht vor, dass die Erfassungseinheit ausgebildet ist, anhand einer Mustererkennung und/oder eines Musterabgleichs und/oder eines Algorithmus das Auftreten von Partikeln und/oder eine Partikelkonzentration und/oder eine Partikeldichte zu ermitteln. Zum Beispiel umfasst die Kameraeinheit eine Kamerasoftware. Beispielsweise ist die Kameraeinheit mit einer Auswerteeinheit gekoppelt. Eine Quantifizierung der Partikel erfolgt durch Ermittlung einer (per Integration ermittelten) Oberfläche, die im Kamerabild von Partikeln bedeckt ist. Anschließend werden erfasste Daten zur partikelbedeckten Oberfläche in Verhältnis zur gesamten erfassten Oberfläche im Rohrinnenraum gesetzt. Insbesondere arbeitet die Kamerasoftware und/oder die Auswerteeinheit beispielsweise mit einem sogenannten Pattern-Matching oder einem lernenden und/oder analytischen Algorithmus, um die Partikel zu klassifizieren und zu quantifizieren. Dabei kann ein Einlernvorgang durchgeführt werden, um den Algorithmus bezüglich einer Klassifizierung zu konfigurieren. Alternativ oder zusätzlich wird ein analytischer Algorithmus eingesetzt, wobei beispielsweise dunkle und nicht dunkle Flächen im Medium ermittelt werden.

In einer Weiterbildung ist die Erfassungseinheit ausgebildet, ermittelte Daten an eine Datenverarbeitungseinheit zu übertragen. Beispielsweise umfasst die Erfassungseinheit Schnittstellen zur drahtlosen Übermittlung der erfassten Daten. In einer weiteren Ausgestaltung ist die Erfassungseinheit in ein Netzwerk eingebunden und liefert permanent Daten über eine mögliche Wasser- und/oder Trinkwasserverschmutzung, die dann beispielsweise von einem Kunden per Applikationssoftware oder mit einer anderen Auswerteplattform abrufbar ist. Der Kunde ist beispielsweise Betreiber des Wasserversorgungssystems. Bei Erfassung von erhöhter Wasser- und/oder Trinkwasserverschmutzung durch gesundheitsschädliche Partikel kann die Erfassungseinheit eine Information ausgeben, dass eine Spülung oder eine anderweitige Reinigung des Mediums und/oder der Rohrleitung veranlasst werden soll. Die ermittelten Daten können zudem an ein akkreditiertes Labor übermittelt werden, um beispielsweise eine Korrelation zwischen Laborergebnis und den mittels der Erfassungseinheit ermittelten Daten durchzuführen.

Des Weiteren betrifft die Erfindung eine Vorrichtung zur Übermittlung von Daten aus einer Rohrleitung, umfassend zumindest eine Messvorrichtung gemäß der vorhergehenden Beschreibung, und zumindest eine mit der Messvorrichtung gekoppelte und/oder koppelbare Datenverarbeitungseinheit, wie einem zentralen Server oder eines mobilen Endgeräts, wobei die Messvorrichtung erfasste Daten und/oder ermittelte Daten an die Datenverarbeitungseinheit drahtlos übermittelt. Die Messvorrichtung übermittelt erfasste Daten und/oder ermittelte Daten kontinuierlich und/oder in vorgebbaren Zeitabständen an die Datenverarbeitungseinheit.

Des Weiteren betrifft die Erfindung eine Rohrleitung mit einem Rohrinnenraum und einem in dem Rohrinnenraum befindlichen Medium, wobei im Rohrinnenraum zumindest eine Messvorrichtung nach obiger Beschreibung angeordnet ist.

Insbesondere ist eine weitestgehend schnelle, effektive und unkomplizierte Überwachung des Rohrinnenraums möglich.

Ausführungsbeispiele der Erfindung werden im Folgenden anhand von Zeichnungen näher erläutert.

Darin zeigen:
- Figur 1: schematisch eine Ausführungsform einer Messvorrichtung mit zumindest einer Erfassungseinheit zur Erfassung von Partikeln in einer Rohrleitung, wobei die Erfassungseinheit in einem Rohrinnenraum der Rohrleitung angeordnet ist, und
- Figur 2: schematisch eine Ausführungsform einer Messvorrichtung mit zumindest einer Erfassungseinheit, umfassend zumindest eine Filtereinheit und eine Kameraeinheit.

Einander entsprechende Teile sind in allen Figuren mit den gleichen Bezugszeichen versehen.

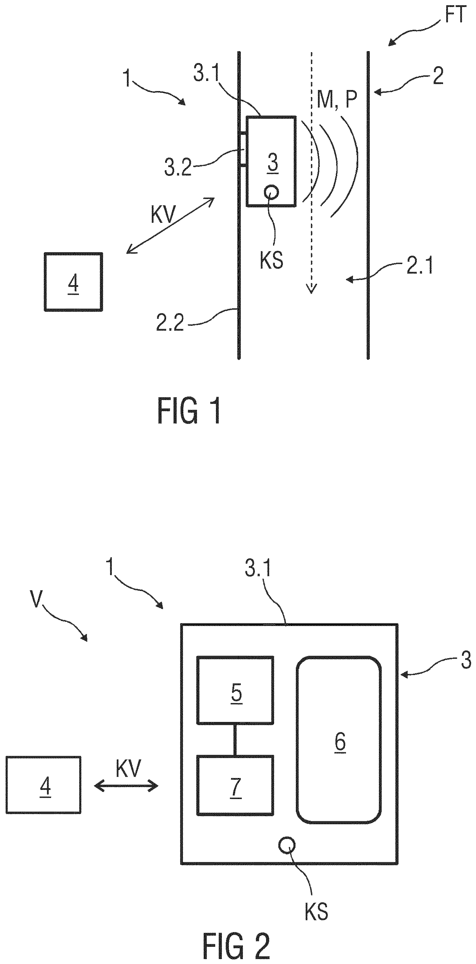

**Figur 1** zeigt schematisch eine Ausführungsform einer Messvorrichtung 1 zur Erfassung von Partikeln P in einer Rohrleitung 2, beispielsweise eines Rohrleitungssystems, insbesondere einer Trinkwasseranlage. Die Rohrleitung 2 ist beispielsweise Teil eines Fluidtransportsystems FT, insbesondere eines Wassertransportsystems.

Die Messvorrichtung 1 umfasst zumindest eine Erfassungseinheit 3, die in einem Rohrinnenraum 2.1 angeordnet ist. Der gezeigte Rohrleitungsabschnitt ist beispielsweise ein Steigleitungsabschnitt oder ein Totleitungsabschnitt der Rohrleitung 2. Die Erfassungseinheit 3 ist beispielsweise als elektronisches Messgerät ausgebildet. Die Erfassungseinheit 3 ist beispielsweise ein in der Rohrleitung 2 stationäres Messgerät.

Der Rohrinnenraum 2.1 weist ein Medium M auf. Das Medium M ist beispielsweise Wasser und/oder Trinkwasser. Beispielsweise durchströmt das Medium M den Rohrinnenraum 2.1. Zum Beispiel steht das Medium M in der Rohrleitung 2.

Die Erfassungseinheit 3 ist ausgebildet, zumindest ein Auftreten unterschiedlicher Partikel P, insbesondere gesundheitsschädlicher Partikel, im Medium M zu erfassen.

Die Messvorrichtung 1 ist als ein in der Rohrleitung 2 stationäres Montagemodul ausgebildet und umfasst ein Gehäuse 3.1, welches die Erfassungseinheit 3 umgibt und kapselt. Das Gehäuse 3.1 kann ein- oder mehrteilig ausgebildet sein. Das Gehäuse 3.1 ist mediumdurchlässig ausgebildet.

Die Erfassungseinheit 3 ist ausgebildet, biologische Partikel P im Wasser und/oder Trinkwasser zu klassifizieren und zu quantifizieren. Zum Beispiel werden unter biologischen Partikel P Pilze und Bakterien, wie Kolibakterien (Escherichia coli, kurz auch E.coli), Legionellen oder andere gesundheitsschädliche Partikel und/oder Schädlinge verstanden. Darüber hinaus ist es möglich, das Medium M auf andere Schadstoffe, wie Korrosionsprodukte, zu überprüfen. Insbesondere sind im Medium M mitgeführte Partikel P mittels der Erfassungseinheit 3 erfassbar. Beispielsweise ist die Erfassungseinheit 3 ausgebildet, Partikelarten zu klassifizieren. Des Weiteren ist die Erfassungseinheit 3 ausgebildet, einen Partikelanteil (Volumenanteil), eine Partikelkonzentration (Volumenkonzentration), ein Volumenverhältnis beispielsweise in Volumenprozent (Vol-%) in Bezug auf ein Volumen des Mediums M und/oder eine Partikeldichte und/oder einen Massenanteil (Masse-%) zu ermitteln.

Die Erfassungseinheit 3 ist beispielsweise ausgebildet, von einem flüssigen Medium M durchströmt zu werden. Alternativ oder zusätzlich ist die Erfassungseinheit 3 ausgebildet, im Medium M zu stehen.

Die Erfassungseinheit 3 ist beispielsweise an einem Innenwandungsabschnitt 2.2 der Rohrleitung 2 befestigt oder lösbar befestigt. Insbesondere ist das Gehäuse 3.1 am Innenwandungsabschnitt 2.2 fixiert. Die Erfassungseinheit 3 ist beispielsweise gegenüber dem Querschnitt der Rohrleitung 2 kleiner ausgebildet. Alternativ korrespondiert eine Abmessung, wie ein Durchmesser, der Erfassungseinheit 3 mit dem Querschnitt der Rohrleitung 2. Das Gehäuse 3.1 kann beispielsweise gegenüber dem Querschnitt der Rohrleitung 2 kleiner ausgebildet sein oder eine Abmessung aufweisen, die der Abmessung der Rohrleitung 2, insbesondere im Querschnitt, entspricht. Beispielsweise umfasst das Gehäuse 3.1 Fixierungselemente 3.2. Die Fixierungselemente 3.2 sind beispielsweise Schraubelemente, Nietelemente, Clips- oder andere Rastelemente. In einer weiteren Ausführungsform sind die Fixierungselemente 3.2 stoffschlüssige Verbindungselemente. Beispielsweise sind die Fixierungselemente 3.2 Schweiß- oder Lötelemente.

Des Weiteren umfasst die Erfassungseinheit 3 eine Filtereinheit 6, die ausgebildet ist, die zu erfassenden Partikel P mit einer Fluoreszenzmarkierung zu versehen. Dabei ist die Erfassungseinheit 3 ausgebildet, fluoreszierende Partikel P zu erfassen. Beispielsweise umfasst die Erfassungseinheit 3 eine Mehrzahl von Teilchen, beispielsweise geeigneten Antikörpern, die mit unterschiedlichen Partikeln P reagieren können. Insbesondere sind die Partikel P durch die Teilchen anregbar, sodass die Partikel P fluoreszieren. Eine chemische Reaktion zwischen Partikel P und Teilchen bewirkt also eine von der Erfassungseinheit 3 erfassbare Fluoreszenz. Beispielsweise ist eine Biofluoreszenz mittels der Erfassungseinheit 3 erfassbar.

Zur Aufnahme der Teilchen umfasst die Erfassungseinheit 3 beispielsweise eine hier nicht näher dargestellte Aufnahmevorrichtung, durch welche das Medium M durchströmen kann.

Zur Partikelerfassung umfasst die Erfassungseinheit 3 beispielsweise einen Sensor.

Beispielsweise ist die Erfassungseinheit 3 ausgebildet ist, anhand einer Mustererkennung und/oder eines Musterabgleichs und/oder eines Algorithmus das Auftreten von Partikeln P und/oder eine Partikelkonzentration und/oder eine Partikeldichte zu ermitteln. Hierfür umfasst die Erfassungseinheit 3 beispielsweise eine Auswerteeinheit 7. Die Auswerteeinheit 7 ist in Form einer Auswerteelektronik, umfassend eine Software und Algorithmen, in die Erfassungseinheit 3 eingebettet.

Des Weiteren erfolgt eine Übertragung erfasster Daten drahtlos. Zum Beispiel ist die Erfassungseinheit 3 ausgebildet, mit stationären oder mobilen Datenverarbeitungseinheiten 4 zu kommunizieren. Beispielsweise weist die Erfassungseinheit 3 eine Anzahl von Schnittstellen, insbesondere Kommunikationsschnittstellen KS, auf. Über die Datenverarbeitungseinheit 4 können mittels der Erfassungseinheit 3 ermittelte und/oder erfasste Daten an Kunden übermittelt werden. Zum Beispiel ist die Erfassungseinheit 3 eingerichtet, erfasste Daten selbst auszuwerten und diese als ermittelte Daten zu übermitteln. Alternativ zusätzlich übermittelt die Erfassungseinheit 3 rein erfasste, nicht ausgewertete Daten.

In einer Weiterbildung ist die Erfassungseinheit 3 in ein Netzwerk eingebunden und liefert permanent Daten über eine mögliche Wasser- und/oder Trinkwasserverschmutzung, die dann beispielsweise von einem Kunden per Applikationssoftware oder mit einer anderen Auswerteplattform abrufbar ist. Der Kunde ist beispielsweise Betreiber des Wasserversorgungssystems. Bei Erfassung von erhöhter Wasser- und/oder Trinkwasserverschmutzung durch gesundheitsschädliche Partikeln P kann die Erfassungseinheit 3 eine Information ausgeben, dass eine Spülung oder eine anderweitige Reinigung des Mediums M und/oder der Rohrleitung 2 veranlasst werden soll. Die ermittelten und/oder erfassten Daten können zudem an ein akkreditiertes Labor übermittelt werden, um beispielsweise eine Korrelation zwischen Laborergebnis und den mittels der Erfassungseinheit 3 ermittelten Daten durchzuführen. Die Datenverarbeitungseinheit 4 ist beispielsweise ein mobiles Endgerät eines Nutzers oder ein zentraler Server. Die Datenverarbeitungseinheit 4 ist beispielsweise eine Recheneinheit. Die Datenverarbeitungseinheit 4 ist beispielsweise ein so genannter Cloud-Speicherplatz. Zum Beispiel ist die Datenverarbeitungseinheit 4 eingerichtet, mit der Erfassungseinheit 3 beispielsweise über ein drahtloses Netzwerk, wie ein globales Netzwerk, Daten der Erfassungseinheit 3 zu empfangen.

Die Messvorrichtung 1 ist eingerichtet, direkt Daten zu erfassen und diese über die Kommunikationsschnittstelle KS zu übertragen. Zum Beispiel ist die Kommunikationsschnittstelle KS als Funkschnittstelle ausgebildet. Die Kommunikationsschnittstelle KS ist ausgebildet, die Erfassungseinheit 3 mit der Datenverarbeitungseinheit 4 zu verbinden. Zum Beispiel werden die Daten an die Datenverarbeitungseinheit 4 übertragen, damit ein Nutzer und/oder ein Kunde zeitnah aktuelle Informationen über das Medium M erhält. Des Weiteren werden die Daten an die Datenverarbeitungseinheit 4 übertragen, damit die Daten beispielsweise von einem Labor erfasst und überprüft werden können.

Die Erfassungseinheit 3 kann eine Steuereinheit umfassen. Alternativ kann eine separate Steuereinheit (nicht näher dargestellt) in der Messvorrichtung 1 vorgesehen sein. Die Erfassungseinheit 3 und die Steuereinheit sind datentechnisch miteinander gekoppelt. Darüber hinaus können/kann die Messvorrichtung 1 und/oder die Erfassungseinheit 3 einen nicht näher dargestellten Speicher zur Speicherung erfasster und/oder ermittelter Daten umfassen. Über die Steuereinheit ist die Erfassungseinheit 3 ansteuerbar. Zudem kann die Erfassungseinheit 3 über die Steuereinheit konfiguriert werden.

Zum Beispiel ist eine Kommunikationsverbindung KV zwischen der Erfassungseinheit 3 und der Datenverarbeitungseinheit 4 zur Datenübertragung herstellbar oder hergestellt. Die Kommunikationsverbindung KV erfolgt über eine oder mehrere Kommunikationsschnittstellen KS. Beispielsweise umgibt und kapselt das Gehäuse 3.1 die Erfassungseinheit 3 und die zumindest eine Kommunikationsschnittstelle KS.

Des Weiteren betrifft die Erfindung eine Rohrleitung 2 mit einem Rohrinnenraum 2.1 und einem in dem Rohrinnenraum 2.1 befindlichen Medium M, wobei im Rohrinnenraum 2.1 zumindest eine Messvorrichtung 1 nach obiger Beschreibung angeordnet ist.

Mittels der Anordnung der Erfassungseinheit 3 direkt im Rohrinnenraum 2.1 ist dieser kontinuierlich/permanent und/oder in vorgebbaren Zeitabständen und/oder bei einer Befehlseingabe vergleichsweise unkompliziert überwachbar. Die Erfassungseinheit 3 ist beispielsweise ausgebildet, in vorgebbaren Zeitabständen Daten zum Zustand des Mediums M zu erfassen. Das in der Rohrleitung 2 transportierte und/oder befindliche Medium M kann insbesondere gegenüber dem Stand der Technik in unkomplizierter Weise auf dessen Hygiene geprüft werden.

**Figur 2** zeigt schematisch eine Ausführungsform einer Messvorrichtung 1 mit zumindest einer Erfassungseinheit 3 zur Erfassung von Partikeln P in einer Rohrleitung 2 eines Fluidtransportsystems FT. Des Weiteren zeigt Figur 2 eine Vorrichtung V zur Übermittlung von Daten aus der Rohrleitung 2.

Die Erfassungseinheit 3 umfasst zumindest eine Kameraeinheit 5 zur Erfassung der im Medium M mitgeführten Partikel P.

Des Weiteren umfasst die Erfassungseinheit 3 zumindest eine Filtereinheit 6. Die Filtereinheit 6 ist vorgesehen, die zu erfassenden Partikel P mit einer Fluoreszenzmarkierung zu versehen. Sobald das Medium M mit der Filtereinheit 6 in Berührung kommt, können die Partikel P mit der Fluoreszenzmarkierung versehen werden. Beispielsweise umfasst die Filtereinheit 6 eine Mehrzahl von Teilchen, insbesondere Antikörpern, die bei Berührung mit insbesondere gesundheitsschädlichen Partikeln P eine chemische Reaktion an einer Partikeloberfläche auslösen, wodurch die Partikel P fluoreszieren.

Die Kameraeinheit 5 ist ausgebildet, die fluoreszierenden Partikel P erfassen zu können. Insbesondere ist die Kameraeinheit 5 konfiguriert, die fluoreszierenden Partikel P im sogenannten VIS-Bereich zu erfassen. Erfasste Daten, wie Bilddaten, werden anschließend ausgewertet. Zum Beispiel ist die Kameraeinheit 5 mit einer Auswerteeinheit 7 gekoppelt.

Die Auswerteeinheit 7 ist in Form einer Auswerteelektronik, umfassend zumindest eine Software und Algorithmen, zur Auswertung der erfassten Daten ausgebildet. Anhand einer Mustererkennung und/oder eines Musterabgleichs und/oder eines Algorithmus sind/ist das Auftreten der Partikel P und/oder eine Partikelkonzentration und/oder eine Partikeldichte erfassbar. Eine Quantifizierung der Partikel P erfolgt durch Ermittlung einer (per Integration ermittelten) Oberfläche, die im Kamerabild von Partikeln P bedeckt ist. Anschließend werden erfasste Daten zur partikelbedeckten Oberfläche in Verhältnis zur gesamten erfassten Oberfläche im Rohrinnenraum 2.1 gesetzt. Insbesondere arbeitet die Auswerteeinheit 7 beispielsweise mit einem sogenannten Pattern-Matching oder einem lernenden Algorithmus, um die Partikel P zu klassifizieren und zu quantifizieren. Dabei kann ein Einlernvorgang durchgeführt werden, um den Algorithmus bezüglich einer Klassifizierung zu konfigurieren.

Die Vorrichtung V umfasst die Messvorrichtung 1 und eine Datenverarbeitungseinheit 4. Die Messvorrichtung 1 und die Datenverarbeitungseinheit 4 sind über eine Kommunikationsverbindung KV, insbesondere einer drahtlosen Kommunikationsverbindung KV, miteinander verbindbar oder verbunden.

### BEZUGSZEICHENLISTE

- 1: Messvorrichtung
- 2: Rohrleitung
- 2.1: Rohrinnenraum
- 2.2: Innenwandungsabschnitt
- 3: Erfassungseinheit
- 3.1: Gehäuse
- 3.2: Fixierungselement
- 4: Datenverarbeitungseinheit
- 5: Kameraeinheit
- 6: Filtereinheit
- 7: Auswerteeinheit

- FT: Fluidtransportsystem
- KS: Kommunikationsschnittstelle
- KV: Kommunikationsverbindung
- M: Medium
- P: Partikel
- V: Vorrichtung

## Patentansprüche

1. Rohrleitung (2) mit einer Messvorrichtung (1) zur Erfassung von Partikeln (P), wobei die Messvorrichtung (1)
- zumindest eine Erfassungseinheit (3) umfasst, die in einem Rohrinnenraum (2.1) der Rohrleitung (2) angeordnet ist, wobei der Rohrinnenraum (2.1) ein Medium (M) aufweist und/oder mit einem Medium (M) durchströmbar ist, und
- wobei die Erfassungseinheit (3) ausgebildet ist, zumindest ein Auftreten unterschiedlicher Partikel (P) im Medium (M) zu erfassen, und
- wobei die Erfassungseinheit (3) zumindest eine Filtereinheit (6) umfasst, die ausgebildet ist, zu erfassende Partikel (P) mit einer Fluoreszenzmarkierung zu versehen.

2. Rohrleitung (2) nach Anspruch 1, wobei die Erfassungseinheit (3) ausgebildet ist, biologische Partikel (P) im Wasser und/oder Trinkwasser zu klassifizieren und zu quantifizieren.

3. Rohrleitung (2) nach Anspruch 1 oder 2, wobei die Erfassungseinheit (3) in einem Steigleitungsabschnitt oder einem Totleitungsabschnitt der Rohrleitung (2) angeordnet ist.

4. Rohrleitung (2) nach einem der vorhergehenden Ansprüche, wobei die Erfassungseinheit (3) ausgebildet ist, fluoreszierende Partikel (P) zu erfassen.

5. Rohrleitung (2) nach einem der vorhergehenden Ansprüche, wobei die Erfassungseinheit (3) zumindest eine Kameraeinheit (5) umfasst.

6. Rohrleitung (2) nach einem der vorhergehenden Ansprüche, wobei die Erfassungseinheit (3) zumindest teilweise lösbar im Rohrinnenraum (2.1) fixiert ist.

7. Rohrleitung (2) nach einem der vorhergehenden Ansprüche, wobei die Erfassungseinheit (3) ausgebildet ist, anhand einer Mustererkennung und/oder eines Algorithmus das Auftreten von Partikeln (P) und/oder eine Partikelkonzentration und/oder eine Partikeldichte zu ermitteln.

8. Rohrleitung (2) nach einem der vorhergehenden Ansprüche, wobei die Erfassungseinheit (3) ausgebildet ist, zumindest ermittelte Daten an eine Datenverarbeitungseinheit (4) zu übertragen.

9. Rohrleitung (2) nach einem der vorhergehenden Ansprüche, wobei die Erfassungseinheit (3) zumindest eine Auswerteeinheit (7) umfasst.

10. Rohrleitung (2) nach einem der vorhergehenden Ansprüche, wobei die Erfassungseinheit (3) eingestellt ist, das Medium (M) kontinuierlich und/oder in vorgebbaren Zeitabständen auf gesundheitsschädliche Partikel (P) zu prüfen.

11. Rohrleitung (2) nach einem der vorhergehenden Ansprüche, wobei die Erfassungseinheit (3) zumindest eine Kommunikationsschnittstelle (KS) aufweist, mittels welcher die Erfassungseinheit (3) zumindest ermittelte Daten überträgt.

12. Vorrichtung (V) zur Übermittlung von Daten aus einer Rohrleitung (2), umfassend
- zumindest eine Messvorrichtung (1) nach einem der vorhergehenden Ansprüche 1 bis 11, und
- zumindest eine mit der Messvorrichtung (1) gekoppelte und/oder koppelbare Datenverarbeitungseinheit (4), wobei die Messvorrichtung (1) erfasste Daten und/oder ermittelte Daten an die Datenverarbeitungseinheit (4) drahtlos übermittelt.

13. Vorrichtung (V) nach Anspruch 12, wobei die Messvorrichtung (1) erfasste Daten und/oder ermittelte Daten kontinuierlich und/oder in vorgebbaren Zeitabständen an die Datenverarbeitungseinheit (4) übermittelt.

## Claims

1. Pipeline (2) comprising a measuring device (1) for detecting particles (P), wherein the measuring device (1)
- comprises at least one detection unit (3), which is arranged in an inner pipe space (2.1) of the pipeline (2), wherein the inner pipe space (2.1) has a medium (M) and/or can be flowed through by a medium (M), and
- wherein the detection unit (3) is designed to detect at least when different particles (P) occur in the medium (M), and
- wherein the detection unit (3) comprises at least one filter unit (6), which is designed to provide particles (P) to be detected with a fluorescent marking.

2. Pipeline (2) according to Claim 1, wherein the detection unit (3) is designed to classify and quantify biological particles (P) in water and/or drinking water.

3. Pipeline (2) according to Claim 1 or 2, wherein the detection unit (3) is arranged in a rising line section or a dead line section of the pipeline (2).

4. Pipeline (2) according to one of the preceding claims, wherein the detection unit (3) is designed to detect fluorescent particles (P).

5. Pipeline (2) according to one of the preceding claims, wherein the detection unit (3) comprises at least one camera unit (5).

6. Pipeline (2) according to one of the preceding claims, wherein the detection unit (3) is at least partially releasably fixed in the inner pipe space (2.1).

7. Pipeline (2) according to one of the preceding claims, wherein the detection unit (3) is designed to determine on the basis of a pattern recognition and/or an algorithm the occurrence of particles (P) and/or a particle concentration and/or a particle density.

8. Pipeline (2) according to one of the preceding claims, wherein the detection unit (3) is designed to transmit at least determined data to a data processing unit (4).

9. Pipeline (2) according to one of the preceding claims, wherein the detection unit (3) comprises at least one evaluation unit (7).

10. Pipeline (2) according to one of the preceding claims, wherein the detection unit (3) is set to test the medium (M) continuously and/or at predeterminable time intervals for particles (P) harmful to health.

11. Pipeline (2) according to one of the preceding claims, wherein the detection unit (3) has at least one communication interface (KS), by means of which the detection unit (3) transmits at least determined data.

12. Device (V) for transmitting data from a pipeline (2), comprising
- at least one measuring device (1) according to one of the preceding Claims 1 to 11, and
- at least one data processing unit (4) coupled and/or couplable to the measuring device (1), wherein the measuring device (1) transmits detected data and/or determined data wirelessly to the data processing unit (4).

13. Device (V) according to Claim 12, wherein the measuring device (1) transmits detected data and/or determined data continuously and/or at predeterminable time intervals to the data processing unit (4).

## Revendications

1. Conduite (2) comprenant un dispositif de mesure (1) destiné à détecter des particules (P), le dispositif de mesure (1) comprenant
- au moins une unité de détection (3) qui est disposée dans un espace intérieur (2.1) de la conduite (2), l'espace intérieur (2.1) comportant un milieu (M) et/ou pouvant être traversé par un milieu (M), et
- l'unité de détection (3) étant conçue pour détecter au moins une survenance de différentes particules (P) dans le milieu (M), et
- l'unité de détection (3) comprenant au moins une unité filtrante (6) qui est conçue pour doter les particules (P) à détecter d'un marqueur à fluorescence.

2. Conduite (2) selon la revendication 1, l'unité de détection (3) étant conçue pour classer et quantifier des particules biologiques (P) dans l'eau et/ou l'eau potable.

3. Conduite (2) selon la revendication 1 ou 2, l'unité de détection (3) étant disposée dans une portion de conduite montante ou une portion de conduite morte de la conduite (2).

4. Conduite (2) selon l'une des revendications précédentes, l'unité de détection (3) étant conçue pour détecter des particules fluorescentes (P).

5. Conduite (2) selon l'une des revendications précédentes, l'unité de détection (3) comprenant au moins une unité formant caméra (5).

6. Conduite (2) selon l'une des revendications précédentes, l'unité de détection (3) étant fixée de manière au moins partiellement amovible dans l'espace intérieur (2.1).

7. Conduite (2) selon l'une des revendications précédentes, l'unité de détection (3) étant conçue pour déterminer la survenance de particules (P) et/ou une concentration de particules et/ou une densité de particules sur la base d'une reconnaissance de formes et/ou d'un algorithme.

8. Conduite (2) selon l'une des revendications précédentes, l'unité de détection (3) étant conçue pour transmettre au moins des données déterminées à une unité de traitement de données (4).

9. Conduite (2) selon l'une des revendications précédentes, l'unité de détection (3) comprenant au moins une unité d'évaluation (7).

10. Conduite (2) selon l'une des revendications précédentes, l'unité de détection (3) étant réglée pour vérifier le milieu (M) en continu et/ou à des intervalles de temps spécifiables pour rechercher des particules nocives (P).

11. Conduite (2) selon l'une des revendications précédentes, l'unité de détection (3) comportant au moins une interface de communication (KS) permettant à l'unité de détection (3) de transmettre au moins des données déterminées.

12. Dispositif (V) destiné à transmettre des données provenant d'une conduite (2), ledit dispositif comprenant
- au moins un dispositif de mesure (1) selon l'une des revendications précédentes 1 à 11, et
- au moins une unité de traitement de données (4) couplée et/ou pouvant être couplée au dispositif de mesure (1), le dispositif de mesure (1) transmettant sans fil des données acquises et/ou des données déterminées à l'unité de traitement de données (4).

13. Dispositif (V) selon la revendication 12, le dispositif de mesure (1) transmettant des données acquises et/ou des données déterminées de manière continue et/ou à des intervalles de temps spécifiables à l'unité de traitement de données (4).
